# EUROPÄISCHE PATENTSCHRIFT

(11) **EP 0 723 531 B1**
(45) Veröffentlichungstag und Bekanntmachung des Hinweises auf die Patenterteilung: **15.10.1997**
(21) Anmeldenummer: 94928865.8
(22) Anmeldetag: 06.10.1994
(51) Int. Cl.: C07C 19/08, C07C 17/04, C07C 17/06, C07C 17/38

(54) **VERFAHREN ZUR HERSTELLUNG VON PENTAFLUORETHAN UND ZUR REINIGUNG VON 1,1,1,2-TETRAFLUORETHAN**
PROCESS FOR PRODUCING PENTAFLUOROETHANE AND FOR PURIFYING 1,1,1,2-TETRAFLUOROETHANE
PROCEDE DE PRODUCTION DE PENTAFLUOROETHANE ET DE PURIFICATION DU 1,1,1,2-TETRAFLUOROETHANE

(30) Priorität: 15.10.1993 DE 4335179
(43) Veröffentlichungstag der Anmeldung: 31.07.1996
(73) Patentinhaber: Solvay Fluor und Derivate GmbH, D-30173 Hannover (DE)
(72) Erfinder: HERKELMANN, Ralf, D-30457 Hannover (DE); RUDOLPH, Werner, D-30559 Hannover (DE); SANDER, Rüdiger, D-31319 Sehnde (DE)
(74) Vertreter: Lauer, Dieter, Dr.
(86) Internationale Anmeldenummer: EP9403299
(87) Internationale Veröffentlichungsnummer: WO9510494

(56) Entgegenhaltungen:
- EP-A- 0 507 458
- GB-A- 785 974
- DATABASE WPI Section Ch, Week 9435, Derwent Publications Ltd., London, GB; Class E16, AN 94-284275 & SU,C,2 009 118 (ASOVICH V S) 15. März 1994
- CHEMICAL ABSTRACTS, vol. 115, no. 7, 19. August 1991, Columbus, Ohio, US; abstract no. 070902, RADECK W ET AL 'Method for removal of unsaturated and/or H-containing impurities from perfluorocarbons or their mixtures' & DD,A,287 478 (AKADEMIE DER WISSENSCHAFTEN DER DDR;FED. REP. GER. ) 28. Februar 1991
- PATENT ABSTRACTS OF JAPAN vol. 017, no. 158 (C-1041) 29. März 1993 & JP,A,04 321 632 (SHOWA DENKO KK) 11. November 1992
- CHEMICAL ABSTRACTS, vol. 103, no. 15, 14. Oktober 1985, Columbus, Ohio, US; abstract no. 122980, 'Octafluoropropane' & JP,A,8 581 134 (SHOWA DENKO K. K.;JAPAN ) 9. Mai 1985

## Beschreibung

Die vorliegende Erfindung betrifft ein Verfahren zur Herstellung von Pentafluorethan und seine Anwendung im Rahmen der Herstellung von 1,1,1,2-Tetrafluorethan mit einem verringerten Gehalt an ungesättigten CF₂-bzw. CHF-Komponenten durch Kontaktieren mit höhervalenten Metallfluoriden.

1,1,1,2-Tetrafluorethan (R134a) und Pentafluorethan (R125) sind bekanntermaßen Ersatzstoffe für vollhalogenierte FCKW. R134a kann beispielsweise als Treibmittel oder als Lösungsmittel verwendet werden. Gemäß einer Herstellungsmethode kann 1,1,1,2-Tetrafluorethan durch Anlagerung von Fluorwasserstoff an Trifluorethylen (R1123) hergestellt werden. Das solcherart hergestellte R134a enthält in geringem Maße Verunreinigungen in Form von ungesättigten C-2-Komponenten mit CHF- bzw. CF₂-Gruppen, insbesondere in Form von Trifluorethylen. Derartige ungesättigte Verbindungen können toxische Wirkung aufweisen. Es ist deshalb wünschenswert, R134a mit einen verringerten Gehalt an derartigen ungesättigten CHF-Komponenten herzustellen. Bei der Herstellung von Pentafluorethan aus CHF₂CH₂F mit CoF₃ fällt, je nach Temperatur, ein Gemisch von CHF₂CHF₂, CF₃CHF₂ und gegebenenfalls C₂F₆ an. Das nach dem Prioritätstag der vorliegenden Anmeldung veröffentlichte russische Patent RU 20 09 118 offenbart die Herstellung eines Gemisches aus Penta- und Hexafluorethan aus Ethylen und CoF₃.

Aufgabe der vorliegenden Erfindung ist es, ein Verfahren zur Umwandlung von ungesättigten C-2-Verbindungen mit CHF- oder CF₂-Gruppe, genannt "ungesättigte CHF-Komponente" bzw. "ungesättigte CF₂-Komponente", anzugeben, mit welchem Pentafluorethan, gegebenenfalls gleichzeitig mit 1,1,1,2-Tetrafluorethan, das von ungesättigten Verbindungen gereinigt ist, hergestellt werden kann. Diese Aufgabe wird durch das Verfahren der vorliegenden Erfindung gelöst.

Erfindungsgemäß erfolgt die Herstellung von Pentafluorethan durch Umsetzung von ungesättigten CHF-Komponenten oder CF₂-Komponenten, die 2 Kohlenstoffatome aufweisen, bei 0 bis 250 °C, vorzugsweise 0 bis 150 °C, insbesondere 0 bis 50 °C, bei Normaldruck oder erhöhtem Druck bis 6 bar (abs.), vorzugsweise im Bereich von Normaldruck bis 4 bar (abs.), unter Kontaktieren mit einem höhervalenten Metallfluorid.

Das Verfahren eignet sich als präparative Methode zur Herstellung von Pentafluorethan (R125). Hierzu geht man, wie gesagt, aus von ungesättigten CHF-Komponenten oder CF₂-Komponenten mit 2 Kohlenstoffatomen. Besonders geeignet als Ausgangsmaterial ist CH₂=CF₂ (erhältlich aus Dichlordifluormethan und Methan beim Leiten über eine Platin-Rhodium-Legierung bei ca. 800 °C, siehe US-A 2,687,440), insbesondere CHF=CF₂ (erhältlich aus Chlortrifluorethylen beim Behandeln mit Wasserstoff in Gegenwart von Pd/C bei 130 bis 135 °C, siehe US-A 2,802,887). Man leitet die gasförmigen Ausgangsverbindungen zweckmäßig in einen Autoklaven, der bereits das Metallfluorid, insbesondere CoF₃, enthält. Bereits bei niedrigen Temperaturen und niedrigen Drücken, z. B. bei Temperaturen von Raumtemperatur bis 40 °C und Normaldruck bis hin zu 4 bar (absolut), bildet sich beispielsweise aus CHF=CF₂ in guter Ausbeute bei erstaunlich hoher Selektivität das gewünschte Pentafluorethan.

Gemäß einer bevorzugten Ausführungsform ist das Verfahren in einem Verfahren zur Herstellung von 1,1,1,2-Tetrafluorethan mit verringertem Gehalt an ungesättigten CHF-Komponenten oder CF₂-Komponenten, wie CHF=CF₂, integriert.

Diese Variante des erfindungsgemäßen Verfahrens dient zur Reinigung von 1,1,1,2-Tetrafluorethan, welches ungesättigte CF₂- bzw. CHF-Komponenten enthält, das man unter den vorgenannten Bedingungen mit einem höhervalenten Metallfluorid kontaktiert und die ungesättigten CF₂- bzw. CHF-Komponenten dabei zu R125 umsetzt. Das resultierende Metallfluorid wird dann vom gereinigten 1,1,1,2-Tetrafluorethan mit einem verringerten Gehalt an ungesättigtem CHF- bzw. CF₂-Komponenten abgetrennt, beispielsweise durch Dekantieren oder Destillieren.

Der Begriff "höhervalente Metallfluoride" umfaßt solche Metallfluoride, z. B. CoF₃ oder AgF₂, die unter Bildung eines niedrigervalenten Metallfluorids Fluor abspalten können und auf diese Weise die Fluorierung von Verbindungen durch Austausch von Wasserstoff, ggf. auch von Chlor gegen Fluor bzw. die Anlagerung von Fluor an Doppelbindungen bewirken. Bevorzugtes höhervalentes Metallfluorid ist CoF₃; dabei bildet sich nach Abspaltung von Fluor CoF₂.

Selbstverständlich kann man das höhervalente Metallfluorid in einer Menge einsetzen, die geringer ist als diejenige Menge, die stöchiometrisch zur Umwandlung der CF₂- bzw. CHF-Komponenten notwendig ist. Der Reinigungseffekt ist hier natürlich entsprechend geringer. Vorzugsweise setzt man das Metallfluorid in der 1- bis 30-fachen Menge derjenigen Menge ein, die stöchiometrisch zur Umwandlung der Verunreinigungskomponenten notwendig ist. Die ungesättigten CF₂- bzw. CHF-Komponenten werden in nichttoxische gesättigte Verbindungen umgewandelt.

Das nach dem erfindungsgemäßen Verfahren hergestellte R125 bzw. das behandelte R134a wird vom verbleibenden Metallfluorid abgetrennt, beispielsweise durch Dekantieren. Für viele Anwendungszwecke ist eine weitere Reinigung nicht notwendig. Gewünschtenfalls kann man R134a von den Umwandlungsprodukten der ungesättigten CHF-Komponenten abtrennen, beispielsweise durch Destillation.

Gemäß einer besonders bevorzugten Ausführungsform wird das R134a so lange nach dem erfindungsgemäßen Verfahren behandelt, bis der Gehalt an ungesättigten CF₂- bzw. CHF-Komponenten auf unter 500 ppm gesunken ist. Durch Wiederholung der Behandlung kann der Gehalt weiter reduziert werden.

Es ist überraschend, daß das erfindungsgemäße Verfahren überhaupt durchgeführt werden kann. Es war nämlich zu erwarten gewesen, daß nicht nur die ungesättigten CF₂- bzw. CHF-Komponenten, sondern auch R125 bzw. das zu reinigende R134a von den höhervalenten Metallfluoriden angegriffen würde. Dies ist jedoch nur in ganz geringem Umfang der Fall. Überraschend ist, daß Perfluorethan allenfalls in Spuren oder, unter drastischen Bedingungen, in geringen Mengen gebildet wird.

Das erfindungsgemäße Verfahren, das batchweise oder kontinuierlich betrieben werden kann, stellt eine sehr einfache Methode dar, R125 herzustellen bzw. den Gehalt an ungesättigten CF₂- oder CHF-Komponenten in R134a abzutrennen. Auch die anschließende Abtrennung vom Fluorierungsreagenz ist sehr einfach durchzuführen. Die entstehenden Reaktionsprodukte der CF₂- bzw. CHF-Komponenten, insbesondere Pentafluorethan, stellen Wertstoffe dar. Das Metallfluorid kann mittels Fluor wieder in ein höhervalentes Metallfluorid überführt und so im Kreislauf geführt werden.

Die Erfindung wird mit folgenden Beispielen weiter erläutert, ohne daß sie in ihrem Umfang eingeschränkt werden soll.

### Beispiel 1:

### Reinigung von R134a bei Raumtemperatur

Ein Gemisch aus 95 GC% 1,1,1,2-Tetrafluorethan und 5 GC% Trifluorethylen wird mit dem 2-fachen der stöchiometrisch benötigten Menge Kobalttrifluorid in einem Autoklaven bei Raumtemperatur und einem Überdruck von 0,5 bar behandelt. Nach vier Tagen ist das enthaltene Trifluorethylen bis auf 300 ppm abgereichert, der Gehalt an Pentafluorethan beträgt 5 GC%. Tetrafluorethen bzw. Perfluorethan sind nur in Spuren nachweisbar. R134a wird unter den gegebenen Bedingungen nur in geringem Umfang zu R125 weiterfluoriert.

### Beispiel 2:

### Reinigung von R134a bei 150 °C

Ein Gemisch entsprechend Beispiel 1 wird mit der 10-fachen stöchiometrischen Menge Kobalttrifluorid mit 0,5 bar Überdruck bei Raumtemperatur autoklaviert. Innerhalb von zwei Stunden wird das System auf 150 °C aufgeheizt und nach Erreichen der Solltemperatur direkt wieder abgekühlt. Das solchermaßen behandelte Gemisch setzt sich aus ca. 90 GC% R134a und 8 GC% R125 zusammen, Trifluorethylen ist nur noch mit 400 ppm enthalten.

### Beispiel 3:

### Reinigung von R134a bei 250 °C

Beispiel 2 wurde wiederholt, jedoch bei der hohen Temperatur von bis zu 250 °C. Man erhält eine abschließende Zusammensetzung von ca. 76 GC% 134a, 21 GC% R125 und 400 ppm 1123 (CF₂= CHF).

### Beispiel 4:

### Präparation von R125

Ein Gemisch mit 90 Vol.-% N₂ und 10 Vol.-% 1123 wurde mit Kobalttrifluorid bei Raumtemperatur und einem Überdruck von 3 bar über drei Tage autoklaviert. Gemäß GC lagen 75 GC% des Produkts in Form von R125 vor.

## Patentansprüche

1. Verfahren zur Herstellung von Pentafluorethan durch Umsetzung von ungesättigten CHF-Komponenten oder ungesättigten CF₂-Komponenten, die zwei Kohlenstoffatome aufweisen, bei 0 bis 250 °C bei Normaldruck oder erhöhtem Druck bis 6 bar (abs.) mit einem höhervalenten Metallfluorid.

2. Verfahren nach Anspruch 1, dadurch gekennzeichnet, daß man bei 0 bis 150 °C, vorzugsweise 0 bis 50 °C arbeitet.

3. Verfahren nach Anspruch 1 oder 2, dadurch gekennzeichnet, daß man bei Normaldruck arbeitet oder bei einem Druck von bis zu 4 bar (abs.).

4. Verfahren nach einem der vorhergehenden Ansprüche, dadurch gekennzeichnet, daß man von CHF=CF₂ ausgeht.

5. Verfahren nach einem der Ansprüche 1 bis 4 zur Reinigung von 1,1,1,2-Tetrafluorethan, welches ungesättigte CHF-Komponenten oder CF₂-Komponenten enthält, unter Kontaktieren desselben unter den vorgenannten Bedingungen mit einem höhervalenten Metallfluorid und Umsetzung der ungesättigten CHF-Komponenten oder CF₂-Komponenten zu Pentafluorethan, zwecks der Herstellung von 1,1,1,2-Tetrafluorethan mit einem verringerten Gehalt an ungesättigten CF₂-Komponenten oder CHF-Komponenten.

6. Verfahren nach Anspruch 5, dadurch gekennzeichnet, daß man 1,1,1,2-Tetrafluorethan behandelt, das mit Trifluorethylen als ungesättigter CF₂- bzw. CHF-Komponente verunreinigt ist, wobei das Trifluorethylen in Pentafluorethan umgewandelt wird.

7. Verfahren nach einem der vorhergehenden Ansprüche, dadurch gekennzeichnet, daß man das höhervalente Metallfluorid in der 1- bis 30-fachen Menge derjenigen Menge einsetzt, welche stöchiometrisch zur Umwandlung der Verunreinigungs-Komponenten in gesättigte Verbindungen notwendig ist.

8. Verfahren nach einem der Ansprüche 5 bis 7, dadurch gekennzeichnet, daß man das 1,1,1,2-Tetrafluorethan destillativ von gebildeten Reaktionsprodukten des höhervalenten Metallfluorids und den ungesättigten CF₂- bzw. CHF-Komponenten abtrennt.

9. Verfahren nach Anspruch 1, dadurch gekennzeichnet, daß man als höhervalentes Metallfluorid CoF₃ einsetzt.

## Claims

1. A method for the preparation of pentafluoroethane by reaction of unsaturated CHF components or unsaturated CF₂ components which have two carbon atoms, at 0 to 250°C at normal pressure or elevated pressure up to 6 bar (absolute) with a higher-valency metal fluoride.

2. A method according to Claim 1, characterised in that one operates at 0 to 150°C, preferably 0 to 50°C.

3. A method according to Claim 1 or 2, characterised in that one operates at normal pressure or at a pressure of up to 4 bar (absolute).

4. A method according to one of the preceding claims, characterised in that the starting point is CHF=CF₂.

5. A method according to one of Claims 1 to 4 for the purification of 1,1,1,2-tetrafluoroethane which contains unsaturated CHF components or CF₂ components, by contacting them under the aforementioned conditions with a higher-valency metal fluoride and reacting the unsaturated CHF components or CF₂ components to pentafluoroethane, for the purpose of the preparation of 1,1,1,2-tetrafluoroethane with a reduced content of unsaturated CF₂ components or CHF components.

6. A method according to Claim 5, characterised in that 1,1,1,2-tetrafluoroethane which is contaminated with trifluoroethylene as unsaturated CF₂ or CHF component is treated, the trifluoroethylene being converted into pentafluoroethane.

7. A method according to one of the preceding claims, characterised in that the higher-valency metal fluoride is used in 1 to 30 times the quantity which is stoichiometrically required for the conversion of the impurity components into saturated compounds.

8. A method according to one of Claims 5 to 7, characterised in that the 1,1,1,2-tetrafluoroethane is separated by distillation from resulting reaction products of the higher-valency metal fluoride and the unsaturated CF₂ or CHF components.

9. A method according to Claim 1, characterised in that CoF₃ is used as the higher-valency metal fluoride.

## Revendications

1. Procédé de production de pentafluoroéthane par la réaction de constituants insaturés comportant un groupe CHF ou de constituants insaturés contenant un groupe CF₂, qui présentent deux atomes de carbone, à 0 jusqu'à 250°C sous la pression normale ou une pression élevée jusqu'à une valeur absolue de 6 bars, avec un fluorure de métal à la valence supérieure.

2. Procédé selon la revendication 1, caractérisé en ce qu'on travaille à une température de 0 à 150°C, de préférence de 0 à 50°C.

3. Procédé selon la revendication 1 ou 2, caractérisé en ce qu'on travaille sous la pression normale ou sous une pression élevée allant jusqu'à une valeur absolue de 4 bars.

4. Procédé selon l'une des revendications précédentes, caractérisé en ce qu'on part de CHF=CF₂.

5. Procédé selon l'une des revendications 1 à 4, pour purifier du 1,1,1,2-tétrafluoroéthane, qui contient des constituants insaturés comportant un groupe CHF ou comportant un groupe CF₂, par mise en contact de ces composés, dans les conditions précitées, avec un fluorure de métal à la valence supérieure, et réaction des constituants insaturés contenant un groupe CHF ou des constituants contenant un groupe CF₂ pour obtenir du pentafluoroéthane, en vue de la production du 1,1,1,2-tétrafluoroéthane ayant une teneur réduite en constituants insaturés contenant un groupe CF₂ ou en constituants contenant un groupe CHF.

6. Procédé selon la revendication 5, caractérisé en ce qu'on traite du 1,1,1,2-tétrafluoroéthane souillé par du trifluoroéthylène comme constituant insaturé contenant un groupe CF₂ ou un groupe CHF, en transformant ainsi le trifluoroéthylène en pentafluoroéthane.

7. Procédé selon l'une des revendications précédentes, caractérisé en ce qu'on utilise le fluorure de métal à la valence supérieure en une quantité représentant 1 à 30 fois la quantité stoéchiométriquement nécessaire pour transformer en composés saturés les constituants présents à titre d'impuretés.

8. Procédé selon l'une des revendications 5 à 7, caractérisé en ce qu'on sépare par distillation le 1,1,1,2-tétrafluoroéthane des produits formés par la réaction du fluorure de métal à la valence supérieure et des constituants insaturés contenant un groupe CF₂ ou CHF.

9. Procédé selon la revendication 1, caractérisé en ce qu'on utilise CoF₃ comme fluorure de métal à la valence supérieure.
